# FASCICULE DE BREVET EUROPEEN

(11) **EP 1 883 800 B1**
(45) Date de publication et mention de la délivrance du brevet: **27.07.2011**
(21) Numéro de dépôt: 06764661.2
(22) Date de dépôt: 22.05.2006
(51) Int. Cl.: G01N 3/06, G01B 5/02, G01B 5/30, G01N 33/24, G01N 3/08

(54) **COLLIER DE MESURE DE LA DEFORMATION LATERALE D'UNE EPROUVETTE LORS D'ESSAIS DE COMPRESSION, NOTAMMENT UNIAXIALE OU TRIAXIALE**
RING ZUR MESSUNG DER SEITLICHEN VERFORMUNG EINES PRÜFLINGS IN UNIAXIALEN ODER TRIAXIALEN KOMPRESSIONSTESTS
COLLAR FOR MEASURING THE LATERAL DEFORMATION OF A TEST PIECE DURING COMPRESSION TESTS, SUCH AS UNIAXIAL OR TRIAXIAL COMPRESSION TESTS

(30) Priorité: 24.05.2005 FR 0505203
(43) Date de publication de la demande: 06.02.2008
(73) Titulaire: UNIVERSITE DES SCIENCES ET TECHNOLOGIES DE LILLE, 59655 Villeneuve d'Ascq (FR)
(72) Inventeur: SECQ, Jean, Uni. des Sciences et Technol. de Lille, 59655 Villeneuve D'Asq (FR)
(74) Mandataire: Boubal, Denis Henri Jacques
(86) Numéro de dépôt international: PCT/FR2006/001165
(87) Numéro de publication internationale: WO 2006/125904

(56) Documents cités:
- FR-A- 2 566 531
- FR-A- 2 663 121
- US-A- 4 579 003
- US-A- 4 905 521
- US-A- 5 483 836

## Description

L'invention se rapporte à un dispositif de mesure de la déformation latérale d'une éprouvette lors d'essais de compression, notamment uniaxiale ou triaxiale.

Elle trouvera une application particulière dans une cellule triaxiale polyvalente d'essais pour géomatériaux d'échantillons de roche, de sol béton et matériaux cimentaires ou de matériaux fabriqués, sur les sites de prélèvement, dans des conditions d'essais des laboratoires.

Ces échantillons, encore appelés éprouvettes, de forme cylindrique, peuvent être soumis à différentes conditions de pression, de chargement, de température, et de drainage, ces paramètres pouvant être contrôlés à l'aide de capteurs de pression, de température, de déplacement interne ou externe.

Les contraintes de pression peuvent être dirigées axialement et/ou sur la face latérale de l'échantillon.

On connaît du document FR-2.566.531 un capteur de déplacement latéral, se présentant sous la forme d'un collier, constitué par une pluralité de rouleaux parallèles, articulés entre eux, et aptes à enserrer l'échantillon maintenu par un système à ressort. Ce collier permet de mesurer la déformation latérale de l'échantillon en mesurant l'écartement des deux derniers rouleaux.

On connaît du document FR-2.663.121 une cellule triaxiale d'essais polyvalente pour géomatériaux. Cette cellule comprend une chambre de pression à l'intérieur de laquelle est placée l'éprouvette cylindrique. Elle comprend au moins un vérin de compression apte à exercer une poussée uniaxiale longitudinalement à l'éprouvette.

Une pression latérale, et plus particulièrement radiale, est exercée sur l'éprouvette en soumettant la face latérale de ladite éprouvette cylindrique à la pression d'un fluide. Un système hydraulique de compensation permet par ailleurs d'équilibrer les efforts longitudinaux et radiaux.

Aussi, l'échantillon est immergé dans un fluide, tel que de l'huile, et est protégé dudit fluide au moyen d'un fourreau se présentant sous la forme d'un manchon constitué par une membrane élastique. Afin de mesurer la déformation latérale de l'échantillon, un collier du type connu, constitué de rouleaux, est placé autour du fourreau en le ceinturant.

Néanmoins, un tel collier s'avère peu satisfaisant, car les rouleaux ont tendance à s'imprimer dans la paroi élastique de la membrane, entravant ainsi leur déplacement par roulement autour de la périphérie du manchon.

En outre, il a été constaté que l'élasticité de la paroi de la membrane fausse la mesure, le collier mesurant la déformation latérale de l'échantillon, mais également la déformation latérale de ladite membrane.

Par ailleurs, le collier connu précité, constitué de rouleaux, s'avère relativement encombrant et nécessite une cellule d'essais dont la chambre de pression est de dimension suffisamment grande, notamment en largeur, pour pouvoir être installée.

On connaît du document US-A-5.483.836 un dispositif permettant la mesure de la déformation latérale d'une éprouvette entre deux points. 11 est constitué par un collier à bascule comprenant deux segments en arc de cercle, articulés l'un à l'autre. Un capteur de type LVDT en combinaison avec une jauge de contrainte permet la mesure de la rotation d'un segment par rapport à l'autre et ainsi la mesure de la déformation de l'échantillon entre deux points de contact.

On connaît du document US-A-4,905.521 un dispositif permettant la mesure de la déformation latérale d'un échantillon, plus particulièrement lors d'une compression triaxiale. Ce dispositif comprend un collier de mesure constitué par une chaîne, permettant une mesure globale de la déformation latérale de l'échantillon, qui coopère avec un fourreau en élastomère qui épouse la paroi latérale de l'échantillon afin de le protéger. Ce dispositif est conforme au préambule de la revendication 1.

On connaît du document US-A-4.579.003 une cellule triaxiale d'essais et l'utilisation de capteur de type LVDT pour les mesures de contrainte sur l'échantillon dans une telle cellule.

Le but de la présente invention est de proposer un collier de mesure de la déformation latérale d'une éprouvette qui pallie les inconvénients précités.

Un autre but de l'invention est de proposer un collier de mesure simple de réalisation et à faible coût de revient.

Un autre but de l'invention est de proposer un collier de mesure apte à coopérer avec un capteur de mesure de déplacement uniaxial standard.

Un autre but de l'invention est de proposer un collier de mesure permettant d'augmenter ou de diminuer la sensibilité de la mesure.

Un autre but de l'invention est de proposer un fourreau de protection pour éprouvette spécifique adapté pour être utilisé avec un collier conforme à l'invention.

D'autres buts et avantages apparaîtront au cours de la description qui va suivre qui n'est cependant donnée qu'à titre indicatif et qui n'a pas pour but de la limiter.

L'invention concerne tout d'abord un dispositif comprenant un collier de mesure de déformation latérale d'une éprouvette, lors d'essais de compression triaxiale, et un fourreau destiné à être utilisé dans une cellule d'essais afin de protéger l'échantillon et coopérant avec le collier, constitué par un manchon élastique, caractérisé en ce que le collier est constitué par un anneau métallique ou un anneau en matériaux composites, apte à enserrer ladite éprouvette en la ceinturant, ouvert, monobloc, et dont les extrémités libres sont écartées d'une distance Δ₁ ledit collier présentant en outre des moyens de mesure de l'écartement Δ desdites extrémités libres de l'anneau, constitués par un palpeur de type transformateur différentiel à variation linéaire, ledit dispositif présentant en outre des moyens mécaniques de renvoi de mesure, le palpeur étant assujetti à l'une des extrémités libres de l'anneau, le doigt du palpeur étant disposé sensiblement perpendiculaire au plan de l'anneau et apte à coopérer avec une came assujettie à l'autre extrémité de l'anneau, ledit manchon présentant dans sa paroi au moins deux points durs, régulièrement répartis sur la circonférence du manchon, pour constituer des points d'appui pour ledit collier, chaque dit point dur étant constitué par un insert d'épaisseur sensiblement égale à l'épaisseur de la paroi du manchon et affleurant des deux côtés de ladite paroi, l'insert présentant en outre des moyens d'enchevêtrement pour le maintien dudit insert au manchon.

L'invention sera mieux comprise à la lecture de la description accompagnée des dessins en annexe parmi lesquels:
- la figure 1 est une vue en perspective d'un collier conforme à l'invention,
- la figure 2 est une vue schématique selon une coupe verticale du collier tel qu'illustré à la figure 1,
- la figure 3 est une vue selon une coupe horizontale, partielle, du collier tel qu'illustré à la figure 1,
- la figure 4 est une vue en perspective d'une éprouvette et d'un fourreau conformes à l'invention,
- la figure 5 est une vue selon la coupe horizontale V-V telle qu'illustrée à la figure 4,
- la figure 6 est une vue de détail du fourneau tel qu'illlustré dans le cadre VI-VI.

L'invention concerne tout d'abord un collier 1 de mesure de déformation latérale d'une éprouvette lors d'essais de compression, notamment uniaxiale ou triaxiale.

On entend par déformation latérale, une déformation de l'éprouvette dans un plan perpendiculaire à l'axe longitudinal de l'éprouvette cylindrique.

Selon l'invention, le collier 1 est constitué par un anneau 2 métallique ou un anneau en matériaux composites, apte à enserrer ladite éprouvette. L'anneau est ouvert.

L'anneau métallique ou en matériaux composites peut être monobloc. II permet d'enserrer l'éprouvette en la ceinturant.

Les extrémités libres 3 de l'anneau 2 sont écartées d'une distance Δ.

Ledit collier 1 présente en outre des moyens 8, 9 de mesure, directement ou indirectement de l'écartement Δ desdites extrémités libres 3 de l'anneau 2, moyens constitués par un palpeur de type transformateur différentiel à variation linéaire.

Le palpeur de type transformateur différentiel à variation linéaire est encore connu sous l'appellation LVDT.

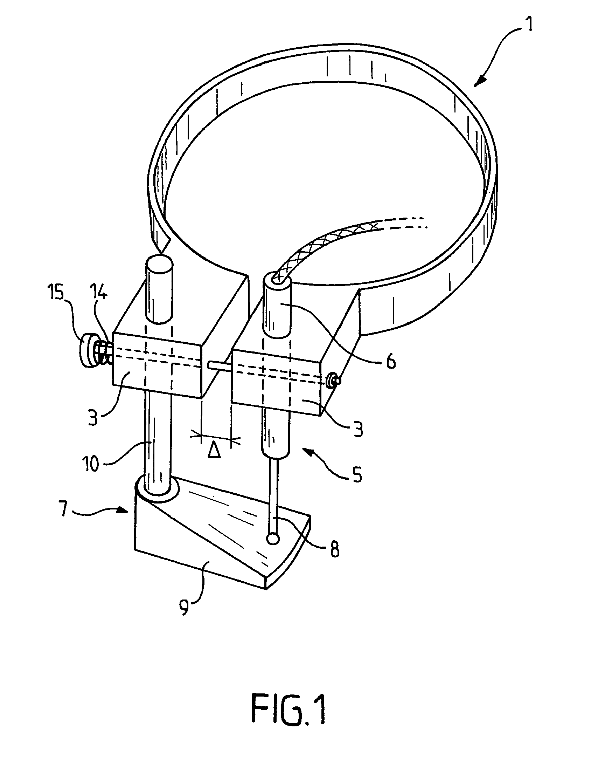

Le collier présente en outre des moyens mécaniques de renvoi de mesure. Aussi, tel qu'illustré à la figure 1, le corps globalement cylindrique du palpeur peut être assujetti à l'une des extrémités libres 3, perpendiculairement au plan du collier permettant ainsi de réduire l'encombrement latérale du dispositif.

Le palpeur 6 est assujetti à l'une des extrémités libres 3 de l'anneau, le doigt 8 du palpeur étant disposé sensiblement perpendiculaire au plan de l'anneau et apte à coopérer avec une came 9 assujettie à l'autre extrémité libre 3 de l'anneau.

La came 9 est assujettie à une extrémité d'une tige 10 de positionnement, et présente, notamment, une forme en portion de cône, dont la génératrice est apte à coopérer avec le doigt 8 du palpeur.

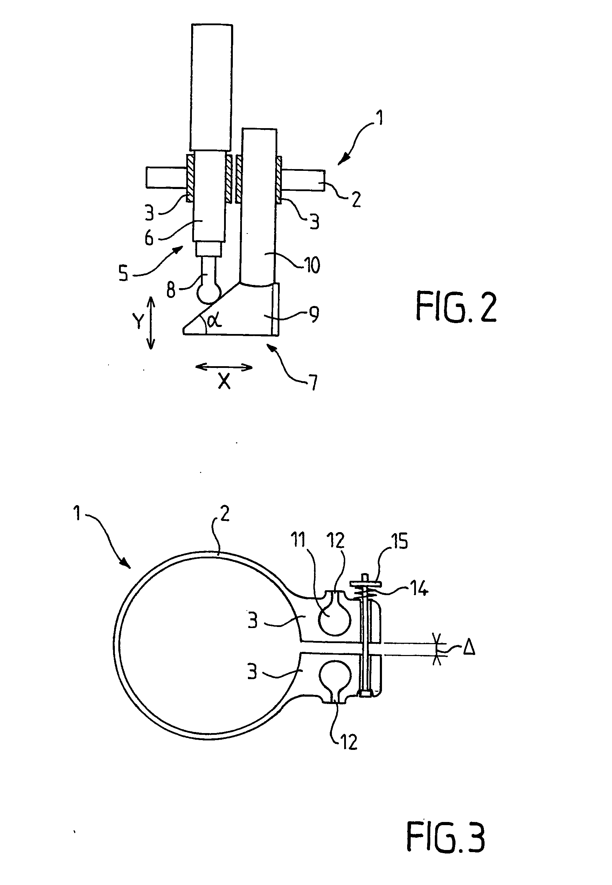

Tel qu'illustré à la figure 1 ou 2, l'axe longitudinal de la tige de positionnement est disposé sensiblement parallèle à l'axe longitudinal du corps du palpeur.

Selon un mode de réalisation, la came présente une pente d'angle α par rapport à un plan perpendiculaire à l'axe longitudinal du doigt 8 du palpeur permettant de régler la sensibilité de la mesure.

Telle qu'illustrée à la figure 2, la génératrice de la portion de cône forme avec un plan parallèle au plan de l'anneau un angle α. Aussi, pour un déplacement relatif X entre les extrémités libres 3 de l'anneau 2, le doigt du palpeur se déplace d'une distance Y = tanα.

Le choix de l'angle α permet alors d'adapter le collier à la course du palpeur, voire même d'augmenter la sensibilité de la mesure. Ainsi, pour un angle supérieur à 45 °, la came joue le rôle d'amplificateur mécanique.

Avantageusement, la came peut être amovible. En fonction du palpeur employé, de l'utilisation ou encore de la sensibilité recherchée, il est ainsi possible de disposer d'un jeu de plusieurs cames présentant des angles α différents.

Selon un mode de réalisation, au moins une extrémité libre 3 de l'anneau 2 présente un alésage traversant 11 perpendiculaire au plan de l'anneau, pour la fixation de la came 9 ou encore pour la fixation du corps du palpeur 6. Ledit alésage présente un alésage orthogonal 12 taraudé et débouchant au niveau dudit alésage traversant, apte à coopérer avec une vis de serrage.

Telles qu'illustrées à la figure 3, les extrémités libres 3 de l'anneau sont constituées chacune par une protubérance de matière, disposées parallèles l'une à l'autre. Lesdits alésages traversant 11 ont des diamètres suffisants pour être traversés par le corps du palpeur 6 ou encore par la tige 10 de positionnement de la came 9. Ces derniers peuvent être ajustés dans lesdits alésages notamment au moyen d'une bague entretoise non illustrée.

Selon un mode de réalisation, les deux extrémités libres 3 de l'anneau sont traversées par une tige de guidage notamment muni de moyens élastiques 14. Cette tige de guidage permet au collier de se déformer de manière contrôlée, notamment sans torsion, afin que lesdites extrémités libres 3 restent maintenues en vis-à-vis.

Les moyens élastiques 14 peuvent prendre la forme d'un ressort en compression prenant appui d'une part sur une extrémité libre 3 de l'anneau 2 et d'autre part sur un écrou 15 vissée à l'une des extrémités filetées de la tige de guidage.

Avantageusement, l'écrou 15 permet le réglage de la force de rappel des moyens élastiques 14.

Cela étant, l'invention concerne également un fourreau 20 destiné à être utilisé dans une cellule d'essais, telle que par exemple celle décrite dans le document FR-2.663.121, afin de protéger une éprouvette 21, notamment minérale, de roche et/ou de sol, béton, matériaux cimentaires, ledit fourreau 20 étant constitués par un manchon 22 élastique, et apte à coopérer notamment avec un collier de mesure conforme à l'invention.

Selon l'invention, le manchon 22 présente dans sa paroi au moins deux points durs 24, régulièrement répartis sur la circonférence dudit manchon, et aptes à constituer des points d'appui pour ledit collier. Le manchon peut être réalisé à partir d'une membrane élastique notamment à partir d'un élastomère. Avantageusement, les points durs sont constitués par des éléments de rigidité bien supérieurs à ladite membrane élastique, et supérieurs à la rigidité de l'éprouvette. Aussi, lors des essais, la déformation desdits éléments constituant les points durs est négligeable devant celle de l'éprouvette.

Le manchon présente, selon un mode de réalisation, quatre points durs régulièrement répartis sur la circonférence dudit manchon à 90°

Selon un mode de réalisation, chaque point dur 24 est constitué par un insert d'épaisseur sensiblement égale au moins à l'épaisseur de la paroi du manchon, et affleurant des deux côtés de ladite paroi. L'insert présente en outre des moyens d'enchevêtrement 25, 26 pour le maintien dudit insert au manchon 22.

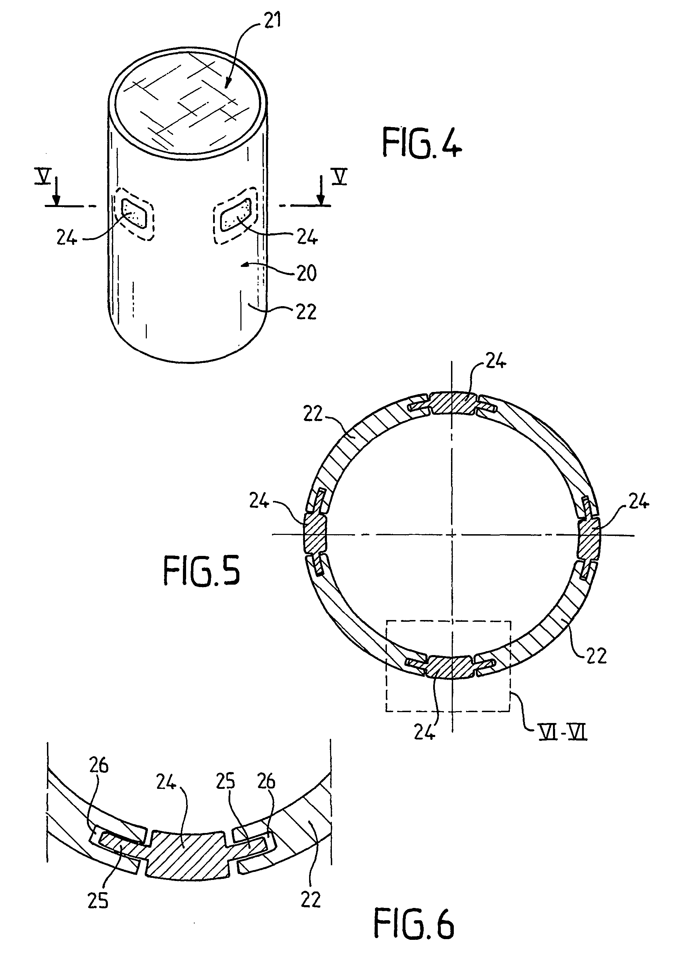

Tels qu'illustrés aux figures 4 à 6, les moyens d'enchevêtrement peuvent être constitués par un tenon latéral 25 apte à s'étendre suivant la paroi du manchon à l'intérieur d'une mortaise 26 pratiquée dans ladite paroi de telle façon à constituer une chicane assurant l'étanchéité du manchon au fluide. Avantageusement, tel qu'illustré à la figure 4, en pointillés, le tenon latéral 25 est continu sur le pourtour de l'insert 24.

Tel qu'illustré à la figure 6, le tenon 25 de l'insert 24 est apte à glisser dans la mortaise 26 pratiquée dans la paroi du manchon lors de la déformation de ce dernier tout en assurant la fonction d'étanchéité.

Selon un mode de réalisation, l'insert est arqué d'un rayon de courbure sensiblement égal au rayon de courbure de la paroi du manchon. Le tenon reprend également ce même rayon de courbure. Le manchon est avantageusement réalisé en silicone matériau souple moulable. Le manchon est avantageusement surmoulé dans un moule où les inserts sont prépositionnés. Avantageusement, l'opération de moulage est réalisée sous vide pour prévenir l'apparition d'inclusions, notamment de bulles.

Naturellement, d'autres modes de mise en oeuvre, à la portée de l'homme de l'art, auraient pu encore être envisagés sans pour autant sortir du cadre de l'invention.

## Revendications

1. Dispositif comprenant un collier (1) de mesure de déformation latérale d'une éprouvette, lors d'essais de compression triaxiale, et un fourreau (20) destiné à être utilisé dans une cellule d'essais afin de protéger l'échantillon et coopérant avec le collier, constitué par un manchon élastique, **caractérisé en ce que** le collier est constitué par un anneau (2) métallique ou un anneau (2) en matériaux composites, apte à enserrer ladite éprouvette en la ceinturant, monobloc, ouvert, et dont les extrémités libres (3) sont écartées d'une distance Δ, ledit collier présentant en outre des moyens (5) de mesure de l'écartement Δ desdites extrémités libres (3) de l'anneau (2), constitués par un palpeur (6) de type transformateur différentiel à variation linéaire, ledit dispositif présentant en outre des moyens mécaniques (7) de renvoi de mesure, le palpeur (6) étant assujetti à l'une des extrémités libres (3) de l'anneau, le doigt (8) du palpeur (6) étant disposé sensiblement perpendiculairement au plan de l'anneau (2) et apte à coopérer avec une came (9) assujettie à l'autre extrémité libre (3) de l'anneau, ledit manchon (22) présentant dans sa paroi au moins deux points durs (24), régulièrement répartis sur la circonférence du manchon, pour constituer des points d'appui pour ledit collier, chaque dit point dur (24) étant constitué par un insert d'épaisseur sensiblement égale à l'épaisseur de la paroi du manchon et affleurant des deux côtés de ladite paroi, l'insert présentant en outre des moyens d'enchevêtrement (25, 26) pour le maintien dudit insert au manchon.

2. Dispositif selon la revendication 1, dans lequel la came (9) est assujettie à une extrémité d'une tige (10) de positionnement et présente une forme en portion de cône dont la génératrice est apte à coopérer avec le doigt (8) du palpeur.

3. Dispositif selon la revendication 1 ou 2, dans lequel la came (9) est amovible.

4. Dispositif selon l'une des revendications 1 à 3, dans lequel au moins une extrémité libre (3) de l'anneau présente un alésage traversant (11), perpendiculaire au plan de l'anneau, pour la fixation de la came (3) ou encore pour la fixation du corps du palpeur (6), ledit alésage présentant un alésages orthogonal (12) taraudé et débouchant au niveau dudit alésage traversant et apte à coopérer avec une vis de serrage.

5. Dispositif selon l'une des revendications 1 à 4, dans lequel les deux extrémités libres (3) de l'anneau sont traversées par une tige de guidage.

6. Dispositif selon l'une des revendications 1 à 5, dans lequel la came présente une pente d'angle α par rapport à un plan perpendiculaire à l'axe longitudinal du doigt 8 du palpeur permettant de régler la sensibilité de la mesure.

7. Dispositif selon la revendication 1, dans lequel les moyens d'enchevêtrement sont constitués par un tenon latéral (25) apte à s'étendre suivant la paroi du manchon à l'intérieur d'une mortaise (26) pratiquée dans ladite paroi de telle façon à constituer une chicane assurant l'étanchéité du manchon au fluide.

8. Dispositif selon la revendication 7, dans lequel l'insert est arqué, d'un rayon de courbure sensiblement égal au rayon de courbure de la paroi du manchon.

9. Dispositif selon l'une des revendications 1 à 8, dans lequel lesdits inserts sont au nombre de quatre.

## Claims

1. Device comprising a collar (1) for measuring the lateral deformation of a test piece, during triaxial compression tests and a shaft (20) intended to be used in a test cell in order to protect the sample and cooperating with the collar, constituted by an elastic sleeve, **characterised in that** the collar is constituted of a metal hoop (2) or a hoop (2) that is made from composite materials, able to clasp said test piece by surrounding it, that is monoblock and, open, and of which the free ends (3) are separated by a distance Δ, said collar further having means (5) for measuring the separation Δ of said free ends (3) of the hoop (2), constituted by a linear variable differential transformer-type probe (6), said device further having des mechanical means (7) for returning the measurement, the probe (6) being fixed at one of the free ends (3) of the hoop, the finger (8) of the probe (6) being arranged substantially perpendicularly to the plane of the hoop (2) and able to cooperate with a cam (9) subjected to the other free end (3) of the hoop, said sleeve (22) having in its wall at least two hard points (24), regularly distributed over the circumference of the sleeve, in order to constitute support points for said collar, each said hard point (24) being constituted by an insert of a thickness substantially equal to the thickness of the wall of the sleeve and flush with the two sides of said wall, the insert further having means for overlapping (25, 26) for the maintaining of said insert in the sleeve.

2. Device according to claim 1, wherein the cam (9) is fixed to one end of a positioning rod (10) and has a shape in the portion of a cone of which the cone distance is able to cooperate with the finger (8) of the probe.

3. Device according to claim 1 or 2, wherein the cam (9) is removable.

4. Device according to one of claims 1 to 3, wherein at least one free end (3) of the hoop has a through-hole (11), perpendicular to the plane of the hoop, for the fastening of the cam (3) or for the fastening of the body of the probe (6), said hole having an orthogonal threaded hole (12) and exiting onto said through-hole and able to cooperate with a clamping screw.

5. Device according to one of claims 1 to 4, wherein the two free ends (3) of the hoop are crossed by a guide rod.

6. Device according to one of claims 1 to 5, wherein the cam has a slope of an angle α in relation to a plane perpendicular to the longitudinal axis of the finger 8 of the probe making it possible to adjust the sensitivity of the measurement.

7. Device according to claim 1, wherein the means of overlapping are constituted by a lateral tenon (25) able to extend according to the wall of the sleeve inside a mortice (26) made in said wall in such a way as to constitute a baffle providing the seal of the sleeve to the fluid.

8. Device according to claim 7, wherein the insert is bowed, by a radius of curvature substantially equal to the radius of curvature of the wall of the sleeve.

9. Device according to one of claims 1 to 8, wherein said inserts are of a number of four.

## Patentansprüche

1. Vorrichtung, umfassend eine Schelle (1) zum Messen der seitlichen Verformung eines Prüfkörpers bei dreiachsigen Druckversuchen und eine Hülse (20), die dazu gedacht ist, in einer Prüfzelle verwendet zu werden, um die Probe zu schützen, und die mit dem Schelle zusammenwirkt, bestehend aus einer elastischen Muffe, **dadurch gekennzeichnet, dass** die Schelle aus einem Metallring (2) oder aus einem Ring (2) aus Verbundmaterialien besteht, der in der Lage ist, den Prüfkörper zu umschließen, indem er ihn umklammert, der einstückig und offen ist, und dessen freie Enden (3) um einen Abstand Δ getrennt sind, wobei die Schelle ferner Mittel (5) zum Messen der Trennung Δ der freien Enden (3) des Ringes (2) aufweist, die aus einem Fühler (6) nach Art eines linear verstellbaren Differentialtransformators bestehen, wobei die Vorrichtung ferner mechanische Mittel (7) zur Rückgabe der Messung aufweist, wobei der Fühler (6) an einem der freien Enden (3) des Ringes befestigt ist, wobei der Finger (8) des Fühlers (6) im Wesentlichen rechtwinklig zur Ebene des Ringes (2) angeordnet ist und in der Lage ist, mit einem Nocken (9) zusammenzuwirken, der an dem anderen freien Ende (3) des Ringes befestigt ist, wobei die Muffe (22) in ihrer Wand mindestens zwei feste Stellen (24) aufweist, die gleichmäßig auf dem Umkreis der Muffe verteilt sind, um Stützpunkte für die Schelle zu bilden, wobei jede feste Stelle (24) aus einem Einsatz im Wesentlichen gleicher Dicke besteht wie die Dicke der Wand der Muffe und mit den beiden Seiten der Wand bündig abschließt, wobei der Einsatz ferner Verblockungsmittel (25, 26) zum Festhalten des Einsatzes an der Muffe aufweist.

2. Vorrichtung nach Anspruch 1, wobei der Nocken (9) an einem Ende eines Stabes (10) zum Positionieren befestigt ist und einen kegelförmigen Abschnitt aufweist, dessen Mantellinie in der Lage ist, mit dem Finger (8) des Fühlers zusammenzuwirken.

3. Vorrichtung nach Anspruch 1 oder 2, wobei der Nocken (9) abnehmbar ist.

4. Vorrichtung nach einem der Ansprüche 1 bis 3, wobei mindestens ein freies Ende (3) des Ringes eine zur Ebene des Ringes rechtwinklige Durchgangsbohrung (11) für die Befestigung des Nockens (3) oder die Befestigung des Körpers des Fühlers (6) aufweist, wobei die Bohrung eine orthogonale Gewindebohrung (12) aufweist, die an der Durchgangsbohrung mündet und in der Lage ist, mit einer Klemmschraube zusammenzuwirken.

5. Vorrichtung nach einem der Ansprüche 1 bis 4, wobei durch die beiden freien Enden (3) des Ringes eine Führungsstange geht.

6. Vorrichtung nach einem der Ansprüche 1 bis 5, wobei der Nocken eine Neigung in einem Winkel α im Verhältnis zu einer Ebene aufweist, die zu der Längsachse des Fingers (8) des Fühlers rechtwinklig ist und es ermöglicht, die Messempfindlichkeit einzustellen.

7. Vorrichtung nach Anspruch 1, wobei die Verblockungsmittel aus einem seitlichen Zapfen (25) bestehen, der in der Lage ist, sich an der Wand der Muffe entlang im Innern eines Zapfenlochs (26) zu erstrecken, das derart in der Wand eingebracht ist, dass es eine Umlenkung bildet, welche die Dichtigkeit der Muffe gegenüber dem Fluid sicherstellt.

8. Vorrichtung nach Anspruch 7, wobei der Einsatz bogenförmig ist, mit einem Krümmungsradius, der im Wesentlichen gleich dem Krümmungsradius der Muffenwand ist.

9. Vorrichtung nach einem der Ansprüche 1 bis 8, wobei es von den Einsätzen vier gibt.
